# EUROPEAN PATENT SPECIFICATION

(11) **EP 3 354 320 B1**
(45) Date of publication and mention of the grant of the patent: **01.09.2021**
(21) Application number: 18153914.9
(22) Date of filing: 29.01.2018
(51) Int. Cl.: A61Q 11/00, A61K 8/04, A61K 8/19, A61C 17/00, A61C 19/06, A61P 1/02, A61P 31/04

(54) **COMPOSITION FOR USE IN ORAL CARE AND RELATED APPARATUS FOR THE TREATMENT OF THE ORAL CAVITY**
ZUSAMMENSETZUNG ZUR VERWENDUNG IN DER ZAHNPFLEGE UND ZUGEHÖRIGE VORRICHTUNG ZUR BEHANDLUNG DER MUNDHÖHLE
COMPOSITION ET APPAREIL POUR LE TRAITEMENT DE LA CAVITÉ BUCCALE

(30) Priority: 31.01.2017 IT 201700010446
(43) Date of publication of application: 01.08.2018
(73) Proprietor: 32 Labs S.r.l., 20123 Milano (IT)
(72) Inventor: SERGI, Chiara, 20123 Milano (IT)
(74) Representative: Tansini, Elio Fabrizio

(56) References cited:
- EP-A1- 2 399 568
- WO-A1-94/26244
- US-A- 5 939 048
- US-A1- 2015 030 546

## Description

The present invention relates to a composition for use in dentistry and an apparatus for the treatment of the oral cavity.

In particular, the present invention refers to an apparatus and to a composition that is dispensed by said apparatus capable of performing a treatment, inside the oral cavity, and in particular on the teeth and gums, of oral hygiene, whitening, prophylaxis and prevention of bacterial formation. As is known **also from documents** EP 2399568 A1**,** WO94/26244 A1**,** US 5939048 A**,** US 2015/030546 A the daily oral cavity hygiene routine is performed using manual or powered toothbrushes to distribute a semi-solid compound (dentifrice usually in the form of a paste) suitable for cleaning the teeth and gums.

With such systems, the compound is distributed on the toothbrush and the bristles are then rubbed against the teeth and partially against the gums, in order to clean the surfaces of the teeth and the gingival sulci and maintain oral hygiene.

This rubbing action results in the removal of any residual food particles, bacterial film and plaque in order to limit the formation of caries and tartar. Certain compounds are also mainly used to perform whitening of the teeth, in combination with the rubbing action of the toothbrush, by removing stains that have formed on the enamel.

Nonetheless, such systems have numerous drawbacks and are not always able to whiten the teeth and maintain oral hygiene in a sufficient and homogeneous manner.

First, the teeth cleaning and whitening operations must be performed using water in order to rinse and thus remove both the paste compound and the residual bacteria that have been removed from the teeth. Therefore, in environments or conditions in which there is no water, such cleaning operations cannot be performed.

Moreover, it is particularly difficult to clean areas of the teeth that are less exposed, and so harder for the bristles to reach.

For example, in the areas between two adjacent teeth or in the region of the molars and generally deep inside the oral cavity it is difficult to use the toothbrush to perform the rubbing action.

Moreover, rubbing too vigorously with the toothbrush and/or for prolonged periods of time could lead to superficial gingival abrasion, causing damage to the gums, and possibly inflammation and bleeding.

This inconvenience is even greater in the case of whitening toothpastes which contain powdered crystals (solids) which therefore tend to rub against the surface of the gums.

Moreover, again in the case of the aforesaid compounds which contain powdered or granular solid elements, the mechanical action of the toothbrush tends to scrape the enamel of the teeth causing progressive damage to the external surface of the teeth.

Also, in the case of dental prostheses, the action of cleaning and whitening the teeth cannot be performed directly in the mouth but the prosthesis must be removed for a dedicated cleaning process.

This procedure has a substantial disadvantage in terms of the time required to perform the daily oral cavity hygiene routine.

To overcome the aforesaid inconveniences, teeth whitening, like the removal of plaque and tartar, is performed professionally using systems and apparatuses used by dentists.

Teeth whitening is thus performed using hydrogen peroxide-based whitening gels, ultraviolet ray laser lamps, whitening strips and even ceramic veneers applied to the vestibular surface of the tooth, treated to resist yellowing.

Devices for whitening teeth for use in a surgery environment are also known in the prior art. These involve the use of a compressor to deliver the whitening powder. In this situation, the operator activates the device, using a respective handpiece, to deliver a flow of whitening powder towards the patient's teeth. The powder is conveyed by the air produced by the compressor at a given operating pressure.

However, the systems used by dentists also have significant drawbacks, mainly associated with the invasive nature of such systems which must therefore be operated by specialised personnel at a surgery.

This results in an important drawback in terms of the cost of each teeth whitening procedure owing to the fact that this procedure cannot be performed autonomously and on a daily basis.

Lastly, another significant drawback of the apparatuses capable of delivering whitening powder is that they require numerous maintenance operations to remove any build-up of powder. After a series of operating cycles, the powder tends to build up and deposit on the delivery ducts thus obstructing them and so preventing correct dispensing of the powder.

In this context, the technical purpose of the present invention is to devise a **use of a** composition and an apparatus for the treatment of the oral cavity that overcome the drawbacks of the prior art solutions mentioned above.

In particular, the purpose of the present invention is to provide a **use of a** composition and an apparatus which perform a treatment action on the teeth and on the gums in a simple manner and without the need for rinsing with water, as defined in the appended set of claims.

In more detail, one purpose of the present invention is to provide a **use of a** composition and an apparatus to clean teeth and gums, by removing bacterial biofilm or other solid residues, uniformly and efficiently in the entire oral cavity, in particular in areas that are difficult to reach during manual cleaning actions.

A further purpose of the present invention is to provide a **use of a** composition and an apparatus that are capable of whitening the teeth efficiently while preserving the integrity of said teeth and of the gums, thus without performing a rubbing action inside the oral cavity.

Yet another purpose of the present invention is to provide an apparatus for use in a home environment, which can be used autonomously and on a daily basis by the user.

A final purpose of the invention is to provide an apparatus that is disposable, structurally simple and which does not require maintenance due to clogging of the composition discharge duct.

The above technical purpose and specified aims are substantially achieved with a **use of a** composition and an apparatus for the treatment of the oral cavity comprising the technical characteristics disclosed in one or more of the appended claims.

Further characteristics and advantages of the present invention will become more apparent from the description that follows of a preferred and non-exclusive embodiment of a **use of a** composition for use in dentistry and an apparatus for the treatment of the oral cavity, represented solely by way of non-limiting example as illustrated in the accompanying drawings in which:

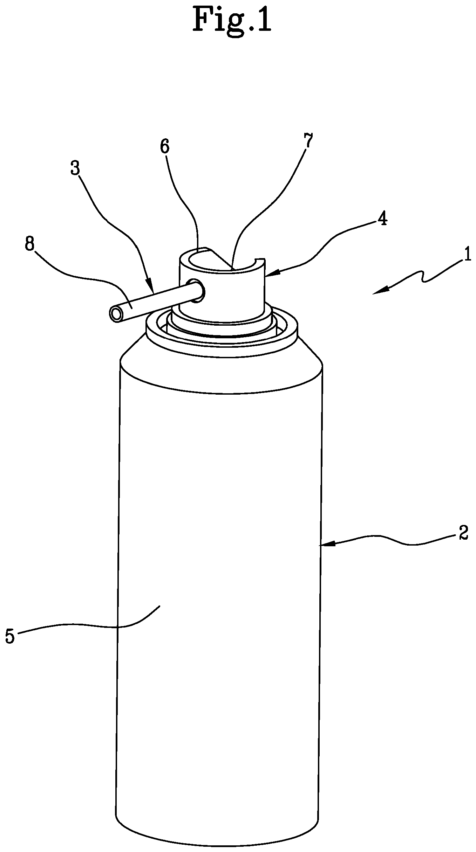

- Figure 1 is a perspective view of an apparatus for the treatment of the oral cavity according to the present invention and in the form of a spray can;
- Figure 2 is a cross-sectional view of the apparatus shown in Figure 1; and
- Figure 3 is a perspective view of a step in the use of the apparatus shown in Figure 1, to deliver a composition onto the teeth and gums of a user.

With reference to the accompanying figures, reference numeral 1 globally denotes an apparatus for the treatment of the oral cavity according to the present invention.

In particular, the apparatus 1 comprises a containing compartment 2 that contains a composition "C" in the liquid state, to which the present invention also relates and which will be described later on in this document.

The apparatus 1 also has a duct 3 for discharging the composition "C", configured to generate a flow "F" of the composition "C" at a given discharge pressure.

The apparatus 1 also comprises a delivery valve 4 interposed between the containing compartment 2 and the discharge duct 3. The valve is switchable between a delivery condition in which it places the compartment 2 in fluidic connection with the duct 3 to permit the passage of a predefined amount of the composition "C", and a closed condition in which it does not permit the passage of the composition "C" between the compartment 2 and the duct 3.

Advantageously, as is clearly illustrated in the accompanying figures, the apparatus 1 is in the form of a spray can.

Consequently, the containing compartment 2 is defined by an internally hollow substantially cylindrical portion 5, preferably made of aluminium. The cylindrical portion 5 which, as illustrated in the accompanying figures, preferably has a circular cross section, may be of any shape so as to define a substantially ergonomic profile.

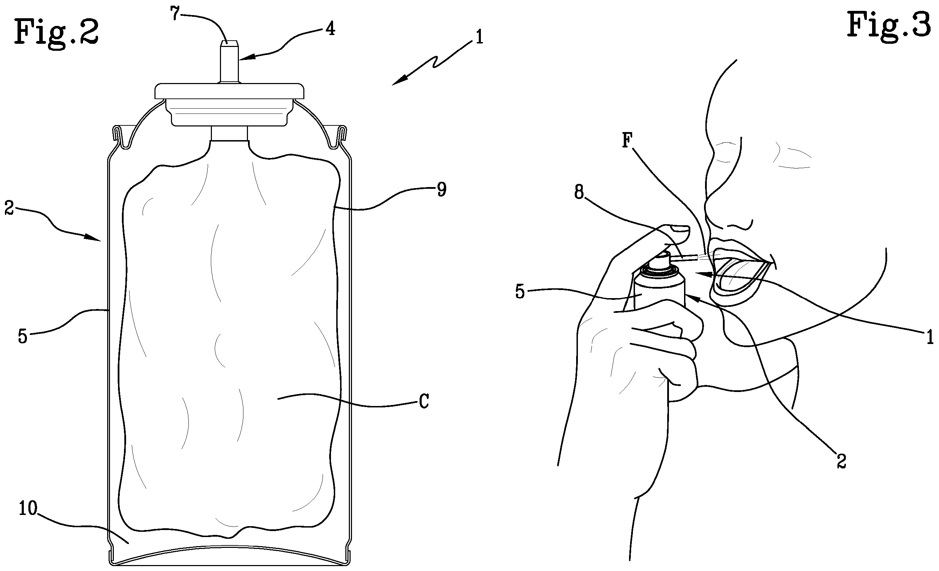

In this way, the user can easily grip the entire spray can 1 to deliver the composition "C" into the oral cavity (Figure 3).

Furthermore, in this situation, the cylindrical portion 5 has an upper end engaged by the valve 4 which has an actuator 6 that can be operated manually to switch said valve 4 to the respective delivery or non-delivery conditions.

Preferably, the actuator 6 has a button 7 that can be pressed by the user, using at least one finger, while gripping the cylindrical portion 5.

The discharge duct 3 comprises a tubular element 8 extending from the valve 4 in a direction crosswise to the longitudinal extension of the cylindrical portion 5.

The tubular element internally defines a flow-through section configured to generate a jet of flow "F" along a respective direction of application and at a pressure of between 8 and 10 bar.

According to a first preferred embodiment illustrated in detail in Figure 2, the compartment 2 internally comprises a bag 9 that contains the composition "C" and is preferably made of a flexible and fluid-tight material. The bag 9 is operationally coupled to the valve 4 to permit the passage of the flow "F" of the composition "C".

Furthermore, the bag 9 is housed inside the cylindrical portion 5 in such a way as to define a chamber 10 comprised between the inside surface of said portion 5 and the outside surface of the bag 9.

The chamber 10 is configured to contain a propellant gas, preferably compressed air, to exert a compressing action on the bag 9.

In other words, when the valve 4 is in the delivery condition, the compressed air compressing the bag 9 presses it so that the composition "C" can be discharged through the tubular element 8.

The air contained in the chamber 10 is compressed to such a pressure as to define, in cooperation with the flow-through section of the tubular element 8, said pressure of between 8 and 10 bar at which the composition "C" is discharged.

The use of this type of spray can 1 (with internal bag 9 to contain the composition) has numerous advantages.

The composition "C" does not come into contact with other propellant gases, and is always inside the bag 9 in a sterile atmosphere.

Furthermore, the use of compressed air, in addition to being environmentally friendly, means the entire contents of the bag 9 are delivered without altering the temperature at which the composition is delivered.

In addition, the composition "C" inside the bag 9 never comes into contact with the oxygen and so said composition has a very long life cycle without the use of any preservatives.

According to an alternative embodiment of the invention, the spray can 1 may be of the type containing a propellant gas mixed with the composition "C".

In that case, the composition "C" is inside the cylindrical portion 5 together with the propellant gas, preferably an inert gas, usually nitrogen. When the valve 4 is switched to the delivery condition, the nitrogen under pressure comes out of the can 1, with the composition, and flows through the tubular element 8.

Also in this case, the pressure of the nitrogen inside the compartment 2, combined with the flow-through section of the tubular element 8, defines the pressure of the flow "F" delivered by the spray can 1 and which is between 8 and 10 bar.

The present description also refers to a composition "C" for use in dentistry, which is applied at a pressure of between 8 and 10 bar.

In particular, the composition "C" comprises approximately 10-15% in weight of sodium bicarbonate (NaHCO₃) and water at approximately 100% in weight.

According to a particularly preferred aspect, the composition "C" may be a saturated solution of sodium bicarbonate stable at the temperature and pressure conditions in which the composition "C" is in the containing compartment 2 of the apparatus 1. The composition "C" is further characterised in that it does not contain suspended solids with particle sizes of more than approximately 10 nm, preferably more than 5 nm, at the temperature and pressure conditions in which the composition "C" is inside the containing compartment 2 of the apparatus 1. Thanks to such characteristics, the composition "C" can be used in dentistry at the aforesaid pressures without causing abrasion of the surface of the teeth and/or irritation of the gums.

According to one aspect, the present description relates to a composition "C" comprising approximately 10-15% in weight of sodium bicarbonate and water at approximately 100% in weight for use in a method for treating the oral cavity in which the composition "C" is applied at a pressure of between 8 and 10 bar.

In particular, the present description refers to a composition "C" comprising approximately 10-15% in weight of sodium bicarbonate and water at approximately 100% in weight for use in a method for whitening the teeth and maintaining oral hygiene in which the composition "C" is applied at a pressure of between 8 and 10 bar.

The water used to prepare the composition "C" may be distilled water or, and preferably, it may be ultrapure water for use in pharmaceutical applications. The composition "C" may also contain further pharmacologically acceptable additives and carriers of sodium bicarbonate.

According to one aspect, the composition "C" may also comprise silver, preferably colloidal silver, which integrates the bactericidal and/or bacteriostatic function of the sodium bicarbonate.

According to one aspect, the composition "C" may further comprise at least one plant extract with a refreshing and/or emollient action, wherein said at least one plant extract may be chosen from among a dry extract, an alcoholic extract, a hydroalcoholic extract, an essential oil or a mixture thereof. Preferably, the composition "C" may comprise at least one essential oil chosen from among peppermint oil, arnica oil, oil of eucalyptus, citronella oil and a mixture thereof.

According to one aspect, the composition "C" may comprise at least one plant extract as described above in an individual concentration of approximately 0.2-2.0% in weight in relation to the total weight of the composition "C".

Preferably, according to one aspect, the composition "C" may comprise at least one substance chosen from among hyaluronic acid and an aloe, chamomile or calendula based substance. According to a particularly preferred aspect, the composition "C" may comprise a mixture of peppermint essential oil and arnica essential oil, in the individual concentrations stated above.

The composition "C" may be prepared by mixing the components in any order, preferably adding the sodium bicarbonate and any additional ingredients to the water, at room temperature (approximately 20°-30°) and atmospheric pressure (approximately 0.1 MPa), and shaking the mixture vigorously.

Advantageously, the composition described above is applied at a pressure of between 8 and 10 bar and for a period of between 5 and 10 seconds.

According to a further aspect, the present description therefore relates to a method for the treatment of the oral cavity comprising at least a step of applying a composition "C" as described above to the teeth and/or gums at a pressure of between 8 and 10 bar, preferably for a period of between 5 and 10 seconds.

It should be noted that the present invention overcomes the problems of the prior art and achieves significant advantages.

First, the action of removing the bacterial biofilm and plaque is performed by the flow "F" without any additional rubbing action on the teeth or gums. The pressure of the flow "F", combined with the nature of the composition "C", guarantees efficient cleaning of the teeth even in the innermost areas of the oral cavity which are difficult for the bristles of the toothbrush to reach. On the contrary, the jet of flow "F" makes it possible to reach even the rearmost molars and gingival sulci, simply by directing the tubular element 8 towards the areas to be cleaned.

Moreover, the action of the flow preserves the integrity of the gums and teeth, eliminating residual food particles from between the teeth and at the same time making the enamel whiter and shinier and remineralising the enamel and thus reducing tooth sensitivity.

The flow "F" does not scratch the gums, reducing and preventing any gingival inflammation.

Furthermore, the use of the apparatus 1, in the form of a spray can, makes it suitable for use at home and thus on a daily basis, without any professional intervention in a surgery environment, to eliminate the proliferation of bacteria that determine the formation of caries.

The sodium hydrogen carbonate, thanks to its mineral properties with their basic pH value, enhances the anti-acid effect of said compound, which is able to raise the pH of the oral cavity to the detriment of the local bacterial flora.

The compound dispensed by the spray can 1 thus exerts a threefold action, that is to say, whitening, toothpaste and mouthwash.

Furthermore, thanks to the natural active ingredients of the composition and the ease of use of the spray can 1, the apparatus can be used by anyone, even in the absence of water for rinsing.

The composition which is an aqueous solution can be removed naturally from the oral cavity or even swallowed as the active ingredient (sodium bicarbonate) is an additive that is widely used in the food industry, and the composition "C" does not contain any chemical additives such as preservatives or colouring agents.

Therefore the apparatus 1 can be used at any time for example after meals to easily and quickly clean the oral cavity and maintain oral hygiene. Repeated use of the apparatus throughout the day ensures the maintenance of good oral hygiene by keeping the breath fresh and giving a lasting sensation of cleanliness and freshness.

Furthermore, the composition "C", which is kept at a controlled pressure, is always in the form of a stable solution. The solid particles are completely dissolved and to not precipitate to the bottom of the compartment 2, so they do not obstruct the valve 4 and/or the tubular element 8.

Furthermore, the pressure at which the composition "C" is delivered always guarantees the total evacuation of the product so that no sedimentation thereof occurs and there is no stagnation i the ducts through which it flows out of the spray can 1.

## Claims

1. Use of a composition comprising 10-15% in weight of sodium bicarbonate and water at 100% in weight, for whitening teeth and maintaining oral hygiene **characterised in that** said composition is applied at a pressure of between 8 and 10 bar.

2. Use as claimed in claim 1, **characterised in that** said pressure is applied for a period of between 5 and 10 seconds.

3. Apparatus for the treatment of the oral cavity, comprising:
- a containing compartment (2) that contains a composition (C) in the liquid state;
- a duct (3) for discharging said composition (C) configured to generate a flow (F) of said composition at a given discharge pressure; and
- a delivery valve (4) interposed between the containing compartment (2) and the discharge duct (3), said valve (4) being switchable between a delivery condition in which it places the compartment (2) in fluidic connection with the duct (3) to permit the passage of a predefined amount of the composition (C), and a closed condition in which it does not permit the passage of the composition (C) between the compartment (2) and the duct (3); **said discharge duct (3) comprising a tubular element (8) extending from the valve (4) and defining a flow-through section for the composition (C) configured to generate a jet of flow (F) along a respective direction of application at a pressure of between 8 and 10 bar;**
**characterised in that** said composition comprises 10-15% in weight of sodium bicarbonate and water at 100% in weight.

4. Apparatus as claimed in the preceding claim, **characterised in that** it is in the form of a spray can (1).

5. Apparatus as claimed in claim 3 or 4, **characterised in that** said containing compartment (2) is defined by an internally hollow cylindrical portion (5); said valve (4) being provided with a manual actuator (6) to switch the valve (4) to the respective conditions and arranged in correspondence with an end portion of the cylindrical portion (5).

6. Apparatus as claimed in the preceding claim, **characterised in that** said cylindrical portion (5) has an ergonomic profile so as to be gripped by a user; said manual actuator (6) having a button (7) that can be pressed while gripping the cylindrical portion (5).

7. Apparatus as claimed in any one of the claims 5 or 6, **characterised in that** said compartment (2) comprises:
- a containing bag (9) that contains the composition (C), coupled to said valve (4) and made of a flexible material; and
- a chamber (10) defined between said cylindrical portion (5) and said containing bag (9); said chamber (10) containing compressed air to define a compressing action on the bag (9).

8. Apparatus as claimed in any one of the claims 5 or 6, **characterised in that** said compartment (2) internally comprises a propellant gas mixed with said composition (C) to enable the delivery of said composition.

## Patentansprüche

1. Verwendung einer Zusammensetzung, umfassend 10-15 Gew.-% Natriumbicarbonat und 100 Gew.-% Wasser, zum Aufhellen der Zähne und zur Aufrechterhaltung der Mundhygiene, **dadurch gekennzeichnet, dass** die Zusammensetzung mit einem Druck zwischen 8 und 10 bar aufgetragen wird.

2. Verwendung nach Anspruch 1, **dadurch gekennzeichnet, dass** der Druck für einen Zeitraum zwischen 5 und 10 Sekunden ausgeübt wird.

3. Vorrichtung zur Behandlung der Mundhöhle, umfassend:
- eine Aufnahmeunterteilung (2), die eine Zusammensetzung (C) in flüssigem Zustand enthält;
- eine Leitung (3) zum Ablassen der Zusammensetzung (C), die ausgebildet ist, um einen Strom (F) der Zusammensetzung bei einem gegebenen Förderdruck zu erzeugen; und
- ein Zuführventil (4), das zwischen der Aufnahmeunterteilung (2) und der Ablassleitung (3) angeordnet ist, wobei das Ventil (4) zwischen einem Zuführzustand, in dem es die Unterteilung (2) in Fluidverbindung mit der Leitung (3) stellt, um den Durchgang einer vordefinierten Menge der Zusammensetzung (C) zu ermöglichen und einem geschlossenen Zustand, in dem es den Durchgang der Zusammensetzung (C) zwischen der Unterteilung (2) und der Leitung (3) nicht ermöglicht, umschaltbar ist; wobei die Auslassleitung (3) ein rohrförmiges Element (8) umfasst, das sich von dem Ventil (4) erstreckt und einen Durchflussabschnitt für die Zusammensetzung (C) definiert, der ausgebildet ist, um einen Strömungsstrahl (F) entlang einer jeweiligen Auftragungsrichtung bei einem Druck zwischen 8 und 10 bar zu erzeugen;
**dadurch gekennzeichnet, dass** die Zusammensetzung 10-15 Gew.-% Natriumbicarbonat und Wasser mit 100 Gew.-% umfasst.

4. Vorrichtung nach dem vorhergehenden Anspruch, **dadurch gekennzeichnet, dass** sie als Sprühdose (1) ausgebildet ist.

5. Vorrichtung nach Anspruch 3 oder 4, **dadurch gekennzeichnet, dass** die Aufnahmeunterteilung (2) durch einen innen hohlen zylindrischen Abschnitt (5) definiert ist; wobei das Ventil (4) mit einer manuellen Betätigungseinrichtung (6) versehen ist, um das Ventil (4) in die jeweiligen Zustände zu schalten, und an einem Endabschnitt des zylindrischen Abschnitts (5) angeordnet ist.

6. Vorrichtung nach dem vorhergehenden Anspruch, **dadurch gekennzeichnet, dass** der zylindrische Abschnitt (5) ein ergonomisches Profil aufweist, um von einem Benutzer ergriffen zu werden; wobei die manuelle Betätigungseinrichtung (6) einen Knopf (7) aufweist, der gedrückt werden kann, während der zylindrische Abschnitt (5) ergriffen wird.

7. Vorrichtung nach einem der Ansprüche 5 oder 6, **dadurch gekennzeichnet, dass** die Unterteilung (2) umfasst:
- einen Aufnahmebeutel (9), der die Zusammensetzung (C) enthält, der mit dem Ventil (4) gekoppelt ist und aus einem flexiblen Material besteht; und
- eine Kammer (10), die zwischen dem zylindrischen Abschnitt (5) und dem Aufnahmebeutel (9) definiert ist; wobei die Kammer (10) Druckluft enthält, um eine Kompressionswirkung auf den Beutel (9) zu definieren.

8. Vorrichtung nach einem der Ansprüche 5 oder 6, **dadurch gekennzeichnet, dass** die Unterteilung (2) innen ein Treibgas enthält, das mit der Zusammensetzung (C) vermischt ist, um die Abgabe der Zusammensetzung zu ermöglichen.

## Revendications

1. Utilisation d'une composition comprenant 10-15 % en poids de bicarbonate de sodium et de l'eau à 100 % en poids, pour le blanchiment des dents et le maintien de l'hygiène buccale, **caractérisée en ce que** ladite composition est appliquée à une pression comprise entre 8 et 10 bars.

2. Utilisation selon la revendication 1, **caractérisée en ce que** ladite pression est appliquée pendant une durée comprise entre 5 et 10 secondes.

3. Appareil pour le traitement de la cavité buccale, comprenant :
- un compartiment de contenance (2) contenant une composition (C) à l'état liquide ;
- un conduit (3), servant à décharger ladite composition (C), configuré pour générer un écoulement (F) de ladite composition à une pression de décharge donnée ; et
- une vanne de distribution (4) interposée entre le compartiment de contenance (2) et le conduit de décharge (3), ladite vanne (4) pouvant commuter entre une condition de distribution dans laquelle elle place le compartiment (2) en raccordement fluidique avec le conduit (3) pour permettre le passage d'une quantité prédéfinie de la composition (C), et une condition de fermeture dans laquelle elle ne permet pas le passage de la composition (C) entre le compartiment (2) et le conduit (3) ; ledit conduit de décharge (3) comprenant un élément tubulaire (8) se prolongeant à partir de la vanne (4) et définissant une section traversante d'écoulement pour la composition (C) configurée pour générer un jet d'écoulement (F) le long d'une direction d'application respective à une pression comprise entre 8 et 10 bars ;
**caractérisé en ce que** ladite composition comprend 10-15 % en poids de bicarbonate de sodium et de l'eau à 100 % en poids.

4. Appareil selon la revendication précédente, **caractérisé en ce qu'**il se présente sous la forme d'un atomiseur (1).

5. Appareil selon la revendication 3 ou 4, **caractérisé en ce que** ledit compartiment de contenance (2) est défini par une partie cylindrique (5) intérieurement creuse ; ladite vanne (4) étant pourvue d'un actionneur manuel (6) pour commuter la vanne (4) dans les conditions respectives et disposée en correspondance avec une partie d'extrémité de la partie cylindrique (5).

6. Appareil selon la revendication précédente, **caractérisé en ce que** ladite partie cylindrique (5) possède un profil ergonomique de manière à être saisie par un utilisateur ; ledit actionneur manuel (6) comportant un bouton (7) pouvant être pressé tout en saisissant la partie cylindrique (5).

7. Appareil selon l'une quelconque des revendications 5 ou 6, **caractérisé en ce que** ledit compartiment (2) comprend :
- un sac de contenance (9) contenant la composition (C), couplé à ladite vanne (4) et constitué d'un matériau flexible ; et
- une chambre (10) définie entre ladite partie cylindrique (5) et ledit sac de contenance (9) ; ladite chambre (10) contenant de l'air comprimé pour définir une action de compression sur le sac (9).

8. Appareil selon l'une quelconque des revendications 5 ou 6, **caractérisé en ce que** ledit compartiment (2) comprend intérieurement un gaz propulseur mélangé à ladite composition (C) pour permettre la délivrance de ladite composition.
